# EUROPEAN PATENT SPECIFICATION

(11) **EP 2 485 745 B1**
(45) Date of publication and mention of the grant of the patent: **03.12.2014**
(21) Application number: 10760902.6
(22) Date of filing: 13.09.2010
(51) Int. Cl.: A61K 35/76, C12N 7/04, C12N 15/86, C12N 15/861, C12N 15/11, C12N 15/63, C12N 15/79

(54) **A composition for creating conditional lethality of virus mutants and for eliminating the viability of an eukaryotic cell**
Zusammensetzung zur Erzeugung von bedingter Letalität von Virusmutanten und zur Eliminierung der Lebensfähigkeit einer eukaryotischen Zelle
Composition pour créer une létalité conditionnelle de mutants de virus et pour éliminer la viabilité d'une cellule eucaryote

(30) Priority: 07.10.2009 EE 200900077
(43) Date of publication of application: 15.08.2012
(73) Proprietor: Tartu Ülikool (University Of Tartu), 50090 Tartu (EE)
(72) Inventor: EL-ANDALOUSSI, Samir, S-170 42 Stockholm (SE); HELLER, Gregory, EE-50106 Tartu (EE); LANGEL, ÜIo, S-113 27 Stockholm (SE); LEHTO, Taavi, EE-50606 Tartu (EE); MERITS, Andres, EE-51017 Tartu (EE); ÜLPER, Liane, EE-50111 Tartu (EE)
(74) Representative: Kahu, Sirje
(86) International application number: PCT/EE2010/000014
(87) International publication number: WO 2011/042029

(56) References cited:
- EP-A1- 1 897 942
- WO-A1-2006/119137
- WO-A2-2005/007832
- WO-A2-2005/023990
- ULPER ET AL: "Construction, properties, and potential application of infectious plasmids containing Semliki Forest virus full-length cDNA with an inserted intron", JOURNAL OF VIROLOGICAL METHODS, ELSEVIER BV, NL, vol. 148, no. 1-2, 3 December 2007 (2007-12-03), pages 265-270, XP022487620, ISSN: 0166-0934, DOI: DOI:10.1016/J.JVIROMET.2007.10.007

## Description

### TECHNICAL FIELD OF THE INVENTION

The present invention relates to the field of controlling gene expression and replication of a virus or virus-based vector introduced into eukaryotic cells. It also relates to the controlling of the rescue of a positive-strand RNA virus or virus based vector from infectious cDNA constructs (layered vectors) inside an eukaryotic cell. Furthermore, the present invention relates to the fields of human and veterinary medicine. In particular, the aim of the invention is to provide a composition for the treatment and prevention of neoplasms, such as tumours, cancers and non-solid tumours.

### BACKGROUND OF THE INVENTION

### APPLICATIONS OF VIRUSES IN TUMOUR TREATMENT

Viruses have evolved specialized molecular mechanisms to transport their genomes efficiently inside the cells they infect. Therefore viral vectors became naturally a tool commonly used by molecular biologists to deliver genetic material into cells. They represent an efficient way of transferring DNA or RNA and expressing recombinant genes. Delivery of genes by a virus is termed transduction and the cells infected by such vector are described as transduced. This process can be performed inside a living organism (in vivo) or in cell culture (in vitro) (US5650309, Wong-Staal et al, 1997). Viral vectors present the advantage of being more efficient in targeting and entering cells than non-viral vectors. They can also be engineered to avoid replication and/or cell destruction. Therefore they represent a relevant choice for an important number of research experiments, cell therapies or gene therapies on animals or humans.

Another important aspect of viral infections is their ability to destroy infected cells and tissues. These properties can be maintained in case of genetically modified viruses, often termed as "viral vectors". The destruction of infected cells can happen by several major mechanisms. Natural mechanisms of destruction of infected cell include:
- via virus-induced cytopathogenic effects (exemplified by virus-induced shutdown of cellular macromolecule synthesis);
- via virus-induced apoptosis of infected cells;
- via action of the immune system (both innate and/or adaptive) of the host.

Virus-induced cell death can also be achieved (or enhanced) by artificial means. For example, cells infected with a virus carrying a conditionally lethal gene, or with a viral vector carrying such gene, can be killed by adding a substrate to the cells which is converted into toxic compound(s) by the product(s) of the conditionally lethal gene (often termed as suicide gene). An example of such gene is (but is not limited to) the thymidine kinase gene (TK) from the herpes simplex virus. The drugs that can be converted into toxic compounds by the product of the suicide gene, are referred to antiherpes virus drugs and are known as aciclovir, ganciclovir etc.

In many cases the destruction of the host cell by a virus can involve enhanced immune reaction against infected tissue. This reaction can target also the components not only encoded by the virus but also encoded by the host cell and tissue. This effect can (and sometimes does) result in auto-immune disorders when the immunological reaction targets and destroys the cells of the organism.

In the context of a healthy organism with normal cells and tissues, these effects are typically referred to as negative effects of the viral infection and termed "virus-induced cytotoxic effects", "virus-induced pathology" etc. However, even then, they can be used to achieve specific and desirable goals such as boosting the efficiency of vaccines (thus, viruses and virus-based vectors can serve as boosters or adjuvant for different types of vaccines). Furthermore, in the context of virus-based therapy of neoplasms, these effects (alone, combined with each other and/or with other mechanisms or therapies) are desirable since they result in the destruction of neoplastic cells and tissues, mobilization of reactions of the organism against these cells and tissues and attracting its immune system against these cells and tissues causing the death of neoplastic cells, tumours etc. (in this case the effect is typically called "breaking the immune tolerance to the tumour"). By these considerations viruses have been long ago envisioned as anti-cancer (anti-neoplastic) agents. Virtually any virus with the ability to infect a neoplastic cell can be engineered to an anti-cancer system. The anti-cancer (anti-neoplastic) effects can be caused by the virus's own genes, their products, or by the viral replication process or, in other cases, caused by transgenes inserted to the viral genome (protein coding sequences, sequences encoding for short interfering RNAs, combinations of different inserted sequences). Anti-neoplastic effect can be caused by one main mechanism or, more often, by a combination of several (naturally occurring or engineered) mechanisms. The list of viruses and virus-based vectors used (or tested) as anti-cancer agents contains viruses from different systemic groups including viruses with DNA genomes (adenoviruses, herpesviruses, parvoviruses etc.) and RNA viruses (including alphaviruses, rhabdoviruses etc.). As it is always observed with viral vectors, each virus based system has their pros and cons and none of them can be referred as universal or ideal.

Unfortunately some of the most common and major inconveniences of the use of viruses or viral vectors in anti-cancer therapy is the difficulty to control in an efficient manner the expression of the transduced gene, the replication process and the tissue-specificity of the virus. These problems are further magnified by the fact that most of the viruses, useful as tools for the treatment of neoplasms, have a broad range of host cells and can (and do) infect also normal cells and tissues causing their destruction which can lead to disease and death. The use of viruses with very narrow host range could represent a solution; unfortunately this approach also suffers from numerous problems:
- typically, there are very few (if any) viruses specific to one single type of host cells. Some preferences are often observed (immune cells for HIV, hepatocytes for HCV, neurons for rabies virus), but almost always there are also other cells the virus can infect and the infection can therefore spread.
- viruses can mutate and one very common result of the mutations is adaptation to new cell types (often, but not always, such adaptation results from the change in receptor specificity of the virus). Typically, a few passages in cell culture are required for the virus to adapt and use new receptors and thus infect new cell type(s).
- even if the narrow host-range virus would be available and its genetic stability achieved (no method known in the art can do it), it would not represent a really useful tool in anti-cancer therapy: this virus will be specific to one type of cancer cell and therefore will have an extremely narrow use. Moreover, since cancer cells are also capable to mutate and are known to change the properties of their surface (such changes are often associated with cancer progression), this type of virus may not be efficient at all.

Therefore the main obstacles in virus-based cancer therapy are:
- achieving cancer-specificity (or, at least, preference) of the virus or virus-based vector;
- keeping its replication/gene expression (and other essential biological properties) intact, so that the virus can kill the targeted cells, break the organism's immune tolerance toward the cancer etc.;
- minimizing the damages caused by the virus to normal cells and to healthy tissues and to avoid, as much as possible, any virus-induced disease.

Unfortunately these goals are often conflicting with each other; for example a virus (vector), which is safe to use, will likely be useless as anti-cancer agent. Thus, realistic anti-cancer agents will likely have side-effects. Therefore, any control mechanism, which can be engineered without causing inactivation of the anti-cancer properties of the vector, will be beneficial.

Making virus specific to cancer cells is typically very challenging. Only a few naturally occurring viruses do have natural specificity to cancer cells. These include (but are not limited to) some parvoviruses (AAV, virus with single-stranded DNA genome), often found in cancer cells and Sindbis virus (virus with positive strand RNA genome) which is reported to have natural tropism to cancer cells. The mechanism(s) of their natural preferences for cancer cells are not well known, but most likely several factors are involved. The naturally occurring factors of cancer-cell preference (if known) could be and are taken into account for the development of viral vectors with artificially enhanced cancer-tissue (cell) specificity.

A number of methods aiming to achieve the cancer tissue specificity of infection by virus or viral vector are known in art. These include (but are not restricted to):
- use of mutant viruses restricted to p53 negative cancer cells;
- substitution of universally active viral promoters with promoters specific to cancer cell or with those, which can be specifically activated by chemical or physical factors;
- re-engineering the region of virions, responsible for binding to a receptor and/or entry into infected cells.
- re-engineering viral regions responsible for interaction with host factors and/or essential for replication of the viral genomes.
- tissue-specific delivery of otherwise non-modified or modified viral systems for example by intra-tumour injection of the virus or by means of tissue-specific delivery systems (nanoparticle, liposomes etc).
- use of conditionally lethal mutants, for example temperature sensitive mutants.

These methods can be used in combination with naturally existing cancer-specificity mechanisms and/or with each other. They can also be combined with other methods increasing safety of viral systems such as the use of conditionally lethal genes, deletion of some genes responsible for pathogenesis from the viral genome and the like.

The nature of the virus itself defines which method(s) could be used and what the possible outcomes would be. The nature of the virus will also define the risks associated with any particular method. The general risks of genetic manipulations of any viruses include the possibility of reversion or compensation of induced changes. The side effects of these changes may lead to the debilitation of the virus and thus to the loss of its ability to cause the death of the cancer cells, and at least, in theory, to the generation of viral strains with unpredicted properties and abilities to cause infection, disease and even death of the patient (and, in the worst case, to the transmission of mutated viruses and new epidemics). Another general problem with these methods is that all of them (with the exception of using delivery systems) require a specific match between the specific cancer cell and the virus used to eliminate its viability. Since cancers (neoplasms) have a vast number of different forms and subtypes it may require the adaptation of the viral system to each and every one of them (or, alternatively, it would require the possibility to modify the viral system so rapidly that they can be matched to any specific type of cancer). It would make these therapies less efficient and more time and resource demanding. In addition, many of these methods (such as the use of cell-specific promoters or regulation elements in virus genomes) are of no use at all in case of viruses with RNA genomes. Such viruses have often excellent oncolytic properties but they do not use any cellular elements for the regulation of their gene expression (instead they use their own replicase and RNA elements recognized by this complex), and accordingly, can not be modified by using such elements. The prior art teaches various methods and compositions for increasing the specificity of oncolytic viruses and vectors. WO2005023990 (Intronn Inc., Mitchell L.G. et al, 2005) describes a conditionally replicative adenovirus producing a pre-trans-splicing molecule (PTM) encoding a light producing enzyme, as well as a PTM carrying a sequence coding for an adenoviral protein essential for viral replication. The principle underlying WO2005023990 is to rely on trans-splicing of the sequence coding for an adenoviral protein essential for viral replication onto a transcript specific for the target cell, in order to target synthesis of said protein and hence replication of said conditional adenovirus into said target cell. WO2005007832 (Cell Genesys Inc., Harding T. et al, 2005) describes a method for targeting conditional replication of an oncolytic virus or vector, by placing essential viral genes under control of a promoter responsive to an inducible transcriptional transactivator, whose coding sequence is under the transcriptional control of a cell type-specific transcriptional response element. WO2006119137 (Univ. North Carolina, Samulski R.J., 2006) describes methods and compositions for regulated expression of nucleic acid at post-transcriptional level, but does not address or hint at cytolytic/oncolytic viruses or vectors, nor at insertion of the intron such as to interrupt the expression of a viral component with a vital function and to result in a lethal phenotype of the virus or vector.

All factors listed above underlay the demand for systems regulating tissue specificity of virus-based therapies. Such systems should, in ideal, be usable with many (preferably with all) viral systems. Such systems should have the possibility to be combined with other regulation systems (both with existing or not yet developed ones) without compromising the anti-cancer properties of the vector. Finally, such system should be easily adaptable to any (or at least to as many as possible) type of cancer cells with minimal need of time and resources.

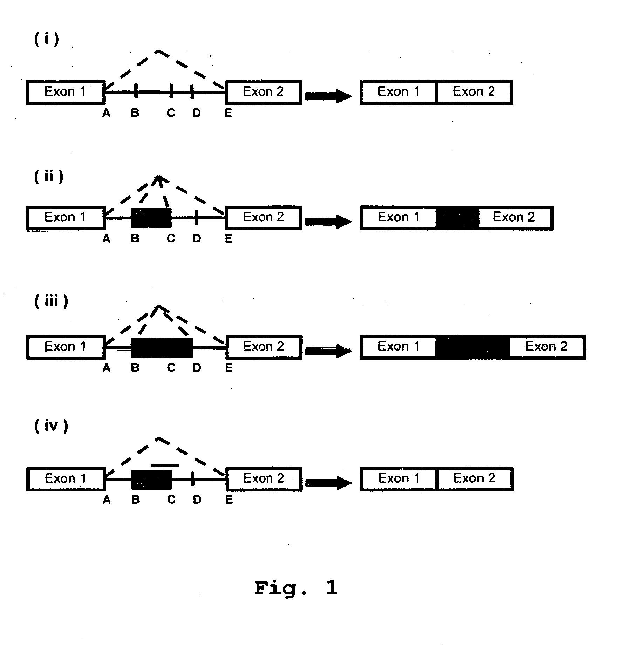

In molecular biology, splicing is a process of modification of an RNA after transcription (pre-mRNA), wherein introns are removed and exons are joined. This is needed for the generation of a typical eukaryotic messenger RNA (mRNA) before it can be transported out from the nucleus and used to produce a correct protein through translation. For many eukaryotic introns, splicing is performed in a series of reactions which are mostly catalyzed by the spliceosome, a complex of small nuclear ribonucleoproteins that exists inside the nucleus of every eukaryotic cell. Any splicing error that adds or removes even one nucleotide of coding sequence will disrupt the open reading frame of an mRNA. For several years now it is known that several genetic diseases arise due to defects in pre-mRNA splicing. Several forms of beta thalassaemia are caused by a single nucleotide mutation within the intronic segments of the human beta-globin gene (Busslinger et al., Beta + thalassemia: aberrant splicing results from a single point mutation in an intron. 1981, Cell 27, 289-298; Jo et al.,A case of beta-thalassemia with a C--T substitution at position 654 of the second intervening sequence of the beta-globin gene. 1992 Intern. Med. 31, 269-272). Within the intron, a 3' splice site, 5' splice site, and branch site are required for splicing. Some of these mutations create an alternative splicing site and/or activate a cryptic branching site that will become preponderantly used during the splicing process and despite the presence of the normal site. The presence of a defective intron results in an incorrectly spliced mRNA and ultimately in an abnormal beta-globin and the disease (Figure 1). It is possible to revert the aberrant splicing with antisense oligonucleotides targeting specific sites on the pre-mRNA molecule (Kole and Dominski, Restoration of correct splicing in thalassaemic pre-mRNA by antisense nucleotides, PNAS, 1993, pp.8673-8677) (Figure 1). These types of splice-corrections have been experimentally shown to result in rescuing the mutated gene expression. The use of this method is envisioned for treatment of such genetic disorders either by temporarily switching the splicing to the normal pathway (US5.916.808, Ryszard Kole et al., 1999) or by causing the reversion in the mutated gene itself and thus leading to permanent removal of the genetic defect (Chin et al., Correction of a splice-site mutation in the beta-globin gene stimulated by triplex-forming peptide nucleic acids. 2008 PNAS, 105:13514-13519).

The use of the antisense oligonucleotides, which block the mutated splicing site and/or activate the splicing using correct splicing sites, is well described (Kole and Dominski, Restoration of correct splicing in thalassaemic pre-mRNA by antisense nucleotides, PNAS, 1993, pp.8673-8677; Svasti et al., RNA repair restores hemoglobin expression in IVS2-654 thalassemic mice 2009, PNAS, 106, 1205-1210). Its use for the correction of the genetic defects and treatment of genetic disorders has been proposed and corresponding IPR protected (US5916808, Ryszard Kole et al., 1999).

The correction of aberrant splicing of the beta-globin intron by a oligonucleotide has been disclosed in EP1897942A1 (Univ. North Carolina, 2008).

### POSITIVE STRAND RNA VIRUS BASED VECTORS (EXAMPLIFIED BY ALPHAVIRUS VECTORS)

Alphaviruses replicate in the cytoplasm of an infected cell. Their naked genomic RNA is infectious (when delivered to the cell, it initiates the infection) and therefore these viruses can be also rescued from plasmids (or other DNA constructs) delivered to the nucleus of the host cells where these constructs are transcribed by the host transcription machinery to produce viral RNAs which will act as genomic RNAs of the virus or the vector. By these properties alphaviruses are similar to all positive-strand RNA viruses and serve as an example for this whole group.

Alphavirus based systems are used for the expression of foreign proteins and they are also promising and important carriers of the antigens against disease-causing agents. Alphaviruses and alphavirus-based vectors can be used also in virus-based anti-cancer therapy (recently reviewed: Atkins G. et al., (2008) Therapeutic and prophylactic applications of alphavirus vectors. Expert Rev Mol Med, 10:e33) The three model alphaviruses, most often serving as vectors, are Sindbis virus (SIN), Semliki Forest virus (SFV) and Venezuelan equine encephalitis (VEE) virus.

**Alphavirus based vectors.** The alphavirus genome is a single-stranded positive polarity RNA of approximately 11.5 kb in length. It encodes two large polyprotein precursors which are co- and post-translationally processed into active processing intermediates and mature proteins (Strauss, J. H. et al., (1994) The alphaviruses: gene expression, replication, and evolution. Microbiol Rev, 58, 491-562). The structural proteins, encoded by the 3' third of the genome, are translated from a subgenomic mRNA generated by internal initiation on the complementary minus-strand template. The nonstructural (ns) polyprotein, designated as P1234, is translated directly from the viral genomic RNA. It is processed into its individual components, the ns-proteins nsP1, nsP2, nsP3 and nsP4. The nsPs have multiple enzymatic and nonenzymatic functions required in viral RNA replication (Kääriäinen, L. et al., (2002) Functions of alphavirus nonstructural proteins in RNA replication. Prog Nucleic Acid Res Mol Biol, 71, 187-222).

Rapid infection cycle, broad host range, high RNA replication rate in the cytoplasm and extreme transgene expression levels have lead to the development of a broad range of alphavirus based vectors (Liljeström, P. et al., (1991) A new generation of animal cell expression vectors based on the Semliki Forest virus replicon. Biotechnology (N Y), 9, 1356-61). The two basic vector types are:
1. replicon vectors
2. genomic vectors.

The genomic vectors of alphaviruses are virus-based vectors which contain a complete set of viral sequences needed for genome replication, structural protein expression and infectious particle (virion) formation and release. They also contain one or more foreign sequences, expressed as part of viral polyproteins or as individual proteins. In alphavirus replicon vectors, the region coding for viral structural proteins has been removed, rendering these vectors unable (on their own) to form virions. Thus, replicons are single-cycle vectors incapable of spreading from infected to non-infected cells. However, productive replication and high level expression of foreign genes can be initiated either by transfecting the replicon RNA into the cytoplasm of the cell, transfecting cell with corresponding layered vector (see below) or by infecting it with packaged alphavirus replicon particles.

The field of the use of alphavirus vectors has been disclosed in several patent applications. The largest number of patents in the field cover the principles of constructing alphavirus based replicon vectors and producing recombinant alphavirus replicon particles (US6190666, Garoff Henrik. et al., 2001); constructing alphavirus-based replicon systems using *in vitro* transcription by RNA polymerases of bacteriophages or by transcription inside of transfected cells (layered systems) as well as packaging cell lines have been described (US6943015, Frolov Ilya. et al., 2005). Another considerable group of inventions describes the use of alphavirus-based vectors (mostly replicons) for specific purposes, most often for gene vaccination (WO2005026316, Liljeström Peter, 2004).

**Alphavirus layered vectors.** Genome of infectious virus or virus-based vector can be released using cellular transcription machinery. In this case the infectious cDNA (icDNA) of the virus or vector should be flanked with eukaryotic transcription elements: with a promoter at the 5' end and a polyA signal at the 3' end. Such constructs have been described for many alphaviruses and alphavirus-based vectors (Dubensky Jr. T.W. et al., (1996) Sindbis virus DNA-based expression vectors: utility for in vitro and in vivo gene transfer. J. Virol. 70, 508-519; Ülper et al. (2008) Construction, properties, and potential application of infectious plasmids containing Semliki Forest virus full-length cDNA with an inserted intron. J Virol Methods. 148:265-270).

Two types of alphavirus-layered vectors with introns are known in the art:
1. Replicon vectors with introns, inserted in vector plasmid in order to increase the infectivity of the plasmids (US5843723, W.Dubensky Thomas et al., 1998). Such introns are always efficiently spliced wild-type introns and they are always inserted in regions, not corresponding to coding regions of alphavirus genomes.
2. Replication competent SFV vector with inserted intron in the region, corresponding to the structural region of alphavirus genome (WO2009/033490; Rausalu Kai et al., 2009). The intron inserted in these constructs is used to stabilize the infectious plasmid in bacterial cells, to increase the yield of plasmid production and to increase the infectivity of the corresponding plasmid for mammalian cells. The intron described in that invention is also by definition a wild-type efficiently spliced intron.

Thus, both methods known in the art describe the use of efficiently spliced introns with aim to increase the stability of the construct and/or increase its infectivity inside of an eukaryotic cell.

It is also known in the art that the insertion of an intron (disruption of the viral sequences by intron-insertion) has been successfully used in the case of several plant and animal infecting positive-strand RNA viruses including potyviruses (Johansen (1996) Intron insertion facilitates amplification of clones virus cDNA in Escherichia coli while biological activity is re-established after transcription in vivo. Proc. Natl. Acad. Sci. USA 93, 12400-12405; Yang et al. (1998) Construction of full-length cDNA clones of lettuce mosaic virus (LMV) and the effects of intron-insertions on their viability in Escherichia coli and their infectivity to plants. Arch. Virol. 143, 2443-2451; Lopez-Moya and Garcia (2000) Construction of a stable and highly infectious intron-containing cDNA clone of plum pox potyvirus and its use to infect plants by particle bombardment. Virus Res. 68, 99-107), tobamoviruses (Marillonnet et al. (2005) Systemic Agrobacterium tumefaciens-mediated trnsfection of viral replicons for efficient transient expression in plants. Nature Biotechnol. 23, 718-723) and coronaviruses (Gonzalez et al. (2002) Stabilization of a full-length infectious cDNA clone of transmissible gastroenterits coronavirus by insertion an intron. J. Virol. 76, 4644-4661). In addition to the stabilization of the corresponding plasmids in *E. coli,* this approach also has a potential to increase the infectivity of cloned sequences and to enhance virus-mediated gene expression (Marillonnet et al. (2005) Systemic Agrobacterium tumefaciens-mediated transfection of viral replicons for efficient transient expression in plants. Nature Biotechnol. 23, 718-723). The possibility to use intron-insertion techniques with cDNA clones of positive-strand RNA viruses from different systemic groups and infecting different hosts supports our claims that our invention, described below, is applicable for all positive strand viruses of eukaryotes.

**Increasing of cell-specificity of positive-strand RNA viruses and corresponding vectors.** Methods to increase the cell specificity of positive stand RNA viruses, known in the art, include:
1. use of temperature sensitive mutations of the virus;
2. use of mutant viruses ability of which to replicate in cells, other than neoplastic cells, has been reduced by introduction of specific mutations in viral sequences altering interactions with host factors and thus making its replication neoplastic cell specific;
3. use of inducible or cell-specific promoters to trigger the viral rescue from layered vector in selected cells;
4. use of conditionally lethal genes inserted in virus genome to block its unwanted replication and spread;
5. use of viruses with mutated (or altered) surface proteins (viral antireceptor) which will infect specifically the targeted cells carrying the corresponding receptor;
6. use of cell- and tissue specific delivery methods, such as intra-tumour injection or delivery of viral genomes (or virions) in complexes with cell- or tissue specific delivery systems.

### DNA VIRUS BASED VECTORS (EXAMPLIFIED BY ADENOVIRUS-BASED VECTOR)

Many viruses infecting humans have a DNA genome and replicate inside the nucleus of the infected cells. This includes members of *Parvoviridae, Circoviridae, Papillomaviridae, Polyomaviridae, Adenoviridae* and *Herpesviridae* families. Despite the drastic difference in the size of their genomes (from 5 kbp to more than 200 kbp) and even their physical forms (single stranded or double stranded DNA; circular or linear DNA) all these viruses transcribe their genes using cellular DNA dependent RNA polymerase II and use splicing for the expression of at least several (often many) of their genes. In addition, several insect viruses, including viruses from the family *Baculoviridae,* can also infect mammalian cells and express genes using splicing. While splicing is used by all viruses from families *Parvoviridae, Papilloma viridae, Polyomaviridae, Adenoviridae* and *Herpesviridae,* the extent of its usage is largely variable. Herpesviruses from *Alphaherpesvirinae* sub-family (such as herpes simplex virus I) are using splicing only to express their immediately early genes; later these viruses suppress splicing. In contrast, splicing is very efficiently used in case of adenoviruses where most, if not all, gene expression units are expressed by means of alternative splicing.

Many genes of viruses with DNA genomes are expressed by means of alternative splicing, a phenomenon which is used to produce more than one mRNA from one gene, and often results in the production of different proteins with different biological functions. Since the proteins resulting from alternatively spliced RNAs have different and often opposite functions for the virus infection cycle, then it is possible to block specifically the expression of proteins, necessary for promoting viral replication cycle and to leave intact (or even activate) the expression of the forms of proteins, which block (inhibit) the progression of the replication cycle of virus.

Genes, regulation of which is most important for construction of cancer-specific vectors, could have different functions. However, the preferred targets belong to the following groups of genes:
1. Virus encoded activators of virus infection. Most frequently these genes encode transcriptional activators, which are required to activate the expression of the rest of viral genes. Often these genes are the first to be expressed in viral infection and are therefore designated as "immediately early" genes. In most cases the block of their expression results in complete block of viral replication.
2. Virus encoded replicase proteins. Small viruses from *Parvoviridae, Papillomaviridae* and *Polyomaviridae* use host polymerase for their replication. However, these viruses do encode at least one protein, involved in the initiation of replication (sometimes the protein is the same as the transcriptional activator described above). Members of *Adenoviridae* and *Herpesviridae* families do encode at least several components of DNA polymerase complex including the enzyme responsible for the synthesis of viral DNA. If the genes encoding one or several of these proteins could be targeted it will result in complete block of viral replication.
3. Additional potential target genes include (but are not limited to) genes encoding for proteins counter-acting with host anti-viral responses, involved in virus-host interactions or, in some cases, in the formation of virions and virus-specific structures.

**Adenoviruses and adenoviral vectors.** Adenoviruses infect different vertebrates and typically cause lytic infection. More than fifty different adenoviruses are known to infect humans. The genome of an adenovirus has a length of ca 35 kbp and encodes around fifty different proteins. The protein encoding regions are arranged as clusters (transcription units) and the expression takes place by using extensive alternative splicing. Most of adenoviruses use cellular protein CAR as their primary receptor and cellular integrins as co-receptor. Both of these molecules are presented on the surface of many different cell types.

Infection of cells by adenovirus depends on many virus-encoded genes, however the most crucial ones are encoded by transcription units E1A/E1B and E2. E1A encodes, using alternative splicing, trans-activator proteins, E1B encodes anti-apoptotic proteins and E2 encodes three replicase proteins of adenovirus. The expression of E1A/E1B genes is triggered by universal (active in many cell types) viral promoter placed close to the left end of linear DNA genome. If these proteins are not expressed or non-functional the adenovirus infection is blocked since E1A is required to activate other promoters of the virus. Similarly, E2 proteins are needed for replication. The productive infection of an adenovirus results in the lysis of the infected cell and in the release of around 10 000 infectious particles.

Adenovirus based expression vectors have been available for decades. The history of the use of adenovirus-based systems as potential anti-cancer therapeutics is the longest among viruses with DNA genome. Several approaches to engineer adenoviruses, suitable for specific infection of cancer cells are known. The examples of those include:
1. Use of an adenovirus with a deletion in E1B region resulting in deficient E1B-55 kDa anti-apoptotic protein (virus is known as ONYX-015). This virus is unable to replicate in cells with intact p53 anti-apoptotic protein since p53 triggered apoptosis of the cells. However, the virus was able to replicate in p53 deficient cancer cells (over 50% of cancers are p53-negative). Thus, the virus was specific to many types of cancers. Despite of good results of pre-clinical trials the mutant virus showed only moderate, if any, anti-cancer effect in clinical trials (Reviewed: Kasuya et al., The potential of oncolytic virus therapy for pancreatic cancer. 2006 Cancer Gene Ther. 12, 725-736).
2. Use of adenovirus where the promoter for E1A/E1B unit is substituted to cancer-specific promoter. These viruses would not be able to activate their gene expression in cells, where the inserted promoter is inactive. Such viral systems have been used for the treatment of several forms of cancer, including prostate cancer (reviewed: Nettelbeck Cellular genetic tools to control oncolytic adenoviruses for virotherapy of cancer. 2008 J.Mol. Med. 86:363-377).
3. Adenoviruses can be engineered to have modified antireceptor and, thus, altered cell-specificity (reviewed: Curiel Strategies to adapt adenoviral vectors for targeted delivery 1999, Ann N Y Acad Sci 886:158-71).
4. Adenoviruses have been used as expression systems for anti-cancer agents such as short hairpin RNAs, inflammatory cytokines etc. (Li; Treatment of prostate cancer cells with adenoviral vector-mediated antisense RNA using androgen-dependent and androgen-independent promoters. 2009. Med. Oncol).
5. Adenovirus particles can be coupled with delivery systems such as homing peptides, antibodies, antibody fragments etc.

Despite of the number of available options (and their combinations) the adenovirus system, ideal or at least sufficiently effective for treatment of any form of cancer has not yet been reported. Nevertheless adenoviral vectors remain among the most promising types of anti-cancer systems based on the viruses with DNA genomes.

Taking into account the facts listed above it can be concluded that the possibility to control adenovirus gene expression represents, in fact, the possibility to control adenovirus infection. The demonstration of the possibility of controlling adenovirus infection stands as proof of concept for controlling infection by all viruses with DNA genomes replicating in the nucleus of the infected cells and any vector systems (including, but not limited to, anti-cancer vectors) based on these viruses.

The closest solutions to the present invention are:
- alphavirus layered vector plasmid pCMV-SFV4, capable to initiate production of infectious virions in cells, transfected with that plasmid. This model is described in patent application WO2009033490 "A method for creating a viral genomic library, a viral genomic library and a kit for creating the same". The sequence of pCMV-SFV4 is disclosed in the patent application.
- an adenovirus vector, commercially sold by several companies, including Stratagene, catalogue number #240009 (AdEasy™ Adenoviral Vector System).

These systems together act as representatives of all RNA-virus based layered vector systems, DNA viruses and DNA virus based vector systems.

Problems to be solved. In case of RNA virus based layered vector, the release of infectious virus genomes and (in case of genomic vectors) particles does take place in any type of cell and in every cell transfected with that vector, thus the release of virions and start (nor time nor the site) of infection can not be controlled. It represents an especially significant problem in case of *in vivo* applications where the start of infection should be controlled and preferably restricted only to selected cell type(s). In case of DNA viruses or vectors based on these viruses, the problem is the lack of control of virus-mediated gene expression and/or viral replication: the viruses and vectors are capable of carrying out these processes in any permissive cell. It represents a problem for several applications of these vectors, especially *in vivo,* when virus-mediated gene expression and/or replication of the virus is desired in some specific, but not in all permissive cells.

### DISCLOSURE OF THE INVENTION

The invention in its broadest sense is as defined in the independent claims.

The solution presented in this invention is the creation of conditionally lethal viral mutants by introducing into the sequence of viral genome or a complementary DNA of a cytolytic virus one or more eukaryotic intron(s), each of which comprises one or more mutations interfering with correct removal of inserted intron(s) by naturally occurring splicing processes. These insertions will result in defects of mRNAs of the viruses and interrupt the expression of viral components with vital functions such as replicases, regulatory proteins or combinations of several of such components. This will result in a lethal phenotype of the virus or vector. The effect of these insertions can be reversed by applying to the selected cells one or more oligonucleotides, modified oligonucleotides or oligonucleotide analogues specific to the intron or introns previously induced in the viral genome or a complementary DNA of a virus, wherein the presence of the oligonucleotide, modified oligonucleotide or oligonucleotide analogue restores the lethality of the virus and eliminates the viability of the infected or transfected cell by restoring correct splicing of the intron and accordingly the biological functionality of the virus.

### DEFINITIONS

In the present invention, several terms have been used, which may comprise, but are not limited to, also variations, specifications and modifications from these obvious to a person skilled in the art, but which in no way restrict the scope of the invention.

"Virus" may mean a virus with DNA or RNA genome, a genetically modified virus, a viral vector, a construct based on a viral vector, a cDNA based vector of an RNA virus (layered vector).

"Viral construct" may mean a viral vector, genetically modified virus, a construct based on a viral vector, a cDNA based vector of an RNA virus (layered vector).

"Layered vector" means complementary DNA (cDNA) construct of a positive strand RNA virus, where the cDNA of the virus or virus-derived vector is inserted into any DNA vector (plasmid, virus genome) and flanked with appropriately positioned transcription signals which can be used by the eukaryotic cell's transcription system to produce the vector's transcripts which will subsequently act as replicating genomes or vectors based on these genomes.

"Oligonucleotides" means DNA or RNA oligonucleotides, PTO oligonucleotides, 2' O-Met oligonucleotides, LNA or PNA oligonucleotides any other modified oligonucleotides or oligonucleotide analogues known in the art. The oligonucleotide may contain modifications in the nucleobases and/or in the sugar residues and/or in the bonds, linking the nulceobase monomers.

"Neoplastic" or "neoplasm" or "tumour" means any type of cells or formations, which exhibit aberrantly increased growth or multiplication properties, benign or malignant, including but not limited to cancer, tumour, non-solid tumour cells.

"Anti-cancer therapy" means therapy, targeted directly or indirectly against any type of neoplasm and neoplastic cells, including solid tumours (cancers, sarcoma) as well as against non-solid tumours.

In order to eliminate the viability of an eukaryotic cell, the present invention provides a solution for creating conditionally lethal viral mutants by introducing into the sequence of a viral genome or a complementary DNA of a cytolytic virus one or more eukaryotic intron(s), each of which comprises one or more mutations interfering with naturally occurring splicing processes; the cells are infected, transfected or transduced with the conditionally lethal viral mutant; and one or more oligonucleotides, modified oligonucleotides or oligonucleotide analogues specific to the intron or introns previously introduced into the viral genome or a complementary DNA of a virus, are delivered or have been delivered into the named eukaryotic cells wherein the presence of the oligonucleotide, and modified oligonucleotide or oligonucleotide analogue restores the lethality of the virus and eliminates the viability of the cell by restoring correct splicing of the intron and accordingly the biological functionality of the virus.

An object of the present invention is the composition for use in elimination of the viability of a eukaryotic cell by simultaneous or consecutive introduction of its components (a) and (b) into a eukaryotic cell.

Said composition comprises (a) a cytolytic virus or a vector originating from a cytolytic virus with one or more naturally occurring, modified or artificially generated eukaryotic intron(s) introduced, which intron(s) comprise(s) one or more mutations interfering with correct removal of said intron(s) by naturally occurring splicing processes, and wherein the insertion of said intron(s) interrupts the expression of a viral component with a vital function and results in a lethal phenotype of said virus or vector, and (b) oligonucleotides, modified oligonucleotides or oligonucleotide analogues specifically able to restore the correct splicing of the introduced naturally occurring, modified or artificially generated eukaryotic intron(s). This kind of composition can be applied to the prevention or treatment of neoplasms in human or veterinary medicine.

The invention represents a universal approach to construct conditionally lethal mutants of viruses with DNA genomes and conditionally inactivated layered vectors of viruses with positive strand RNA genomes. In case of viruses with DNA genomes the conditionally lethal phenotype is created by the introduction of an aberrantly spliced intron or introns of whatever types and origin into regions, encoding factors, crucial for the viral infection cycle. Such intron or introns can be inserted into any position of the gene; they also can be used for substitution of natural introns of viruses. The aberrantly spliced introns can also be created from native introns of the virus by introduction of one or more mutations in the sequence of these introns. Aberrantly spliced introns can also be used to change the expression levels of different mRNAs produced by alternative splicing. Thus the invention comprises any design of cytolytic viral constructs, vectors and viruses which are created by the use of altered splicing of an introduced eukaryotic intron which results in non-infectious or debilitated phenotype of the virus and of oligonucleotide(s) able to restore the correct splicing of the introduced eukaryotic intron(s). The invention also comprises any combination of approaches described above and recited in the claims with any other method(s) used for generation of cancer-cell specific viral vector or virus (or virus or vector, incapable for replication in non-cancer cells).

In case of viruses with positive-strand RNA genomes the conditionally inactivated phenotype of the layered vector is created by the introduction of aberrantly spliced intron or introns of whatever types and origin in regions, corresponding to the sequence encoding for replicase protein of the viruses. Alternatively, the invention covers also any layered vectors, which have been made non-infectious by insertion of aberrantly spliced intron or introns into positions which will result in disruption of the production of the replicase by transcripts, produced by layered vector in the nucleus of the cell. The invention also covers any combination of approaches described above and recited in the claims with any other method(s) used for generation of cancer-cell specific layered vectors or viral genomes, rescued from such vectors.

The rescue of the conditionally lethal phenotype in case of DNA viruses (and vectors based on these viruses) or rescue of the infectivity of transcripts synthesized in cells from layered vectors of RNA viruses by applying specific cofactor(s) is an essential part of the infection. Such cofactor represents an antisense splice-switch oligonucleotide, combination of several splice-switch oligonucleotides or combination of splice-switch oligonucleotide(s) with splicing enhancer oligonucleotide(s). The cofactor is delivered to the cells, in which virus replication (or rescue from layered vectors) is needed to be activated by any methods used for oligonucleotide delivery, including cell (such as cancer cell) specific delivery of such oligonucleotides.

As the principal aim of the invention, a selective mode of eliminating the viability of an eukaryotic cell has been created. This is being achieved by large-scale infection of the cells, preferably of an affected organism with a neoplastic disorder, including tumours, cancer, non-solid neoplasms, with the viral construct with the properties described above, and delivering the specific oligonucleotides to the desired type of cells. Accordingly, the introduction of the specific oligonucleotide will enable the replication of the viral construct and thus the condition of lethality is achieved for the virus-infected cells, wherein the splice-switch oligonucleotide has been delivered. Such delivery of the oligonucleotides may take place simultaneously with the viral infection, or consecutively either before the viral infection or after the viral infection; cofactor consisting of splice-switch oligonucleotide(s) can also be delivered repeatedly at different times.

The main benefit of the proposed technology is its universal nature. It can be used with different viral systems including layered vectors based on viruses with positive strand RNA genomes and viruses with DNA genomes, which replicate inside the nucleus. However, it must be taken into account that the use of the proposed technology will have different impacts on these systems, namely:
1. In case of viruses with positive strand RNA genomes, the only event which can be controlled by the proposed technology is the release of the infectious RNA genome from the DNA based layered vectors. Thus, this is the only event that can be made specific to the targeted cells. After the genomic RNA is released and the replication is initiated in the cytoplasm of targeted cell, no downstream event would be affected by splice switch oligonucleotide since the RNA genome no longer contains an intron nor does it require any splicing. The progeny of the virus (if there would be any, depending from vector design), released from the cells where viral rescue was initiated, will replicate and continue the infection irrespective to the presence or absence of the splice-switch oligonucleotide.
2. In case of DNA viruses the aberrantly spliced intron or introns remain a permanent part of the viral genome. Accordingly, such viruses as well as their progeny will remain dependent from the presence of the splice-switch oligonucleotide. In case of absence (or removal) of splice-switch oligonucleotide, the progeny of such virus is not capable to proceed with infection.

Taking into account the differences between these systems, vectors from positive-strand RNA viruses (on the example of alphavirus vectors) and DNA viruses (on the example of adenovirus vectors) were described above. In provided examples the usability of the technology for both of these systems has been shown. Accordingly, it has been concluded (and claimed) that the developed technology is applicable for all viruses and vector systems based on the viruses belonging to the positive strand RNA viruses or to the DNA-genomic viruses replicating in the nucleus of an infected cell.

Accordingly, the virus of the invention may be a DNA virus. Also, the virus can mean a viral construct, which may be a layered vector containing complementary DNA of an RNA-virus or a construct originating from an RNA virus. In a preferred embodiment, the virus is an alphavirus or a vector based on an alphavirus. In a most preferred embodiment, the virus is Semliki Forest Virus. In another preferred embodiment, the virus is an adenovirus or a vector based on an adenovirus.

In an embodiment of the invention, the eukaryotic intron may be any eukaryotic intron with naturally occurring nucleotide sequence, artificially generated intronic nucleotide sequence or a modified nucleotide sequence of an eukaryotic intron.

In a preferred embodiment, the eukaryotic intron is the second intron of human beta-globin gene with T to G substitution at position 705 or with C to T substitution at position 654. In a most preferred embodiment, the eukaryotic intron is the second intron of human beta-globin gene with T to G substitution at position 705 and with C to T substitution at position 654.

This approach will enable selective elimination of the viability of neoplasms, tumours, cancers. Accordingly, it has a large scale of applications in human medicine and in veterinary medicine.

### BRIEF DESCRIPTION OF THE FIGURES

Fig.1 shows the different splicing events occurring with and without mutations causing aberrant splicing of the pre-mRNA. Boxes represent exons and heavy lines represent introns. The small bar represents a splice-switch oligonucleotide. The dashed lines correspond to splicing mechanisms and the bold arrows to the splicing event. The black boxes represent the portion of intron that remains in the aberrantly spliced mRNA molecule. The letters A, B, C, D and E stage for important splicing sites. A and E are respectively the splice-acceptor and the splice-donor site that are used in a wild-type situation. The intron is totally removed between site A and E during the pre-mRNA maturation (i). Site C and D are mutations (by e.g. thalassaemic mutation 654 and 705) activating a cryptic splice-acceptor site, here named B. The mutation points C and D become cryptic splice-donor sites and a portion of intron is included in the matured mRNA molecule (ii) and (iii). If an oligonucleotide specific to the site C is present, it will hybridize to the pre mRNA and block the aberrant splicing process. As a result of this splice-switch sites A and E are used for splicing and a wild-type mRNA will be produced (iv).

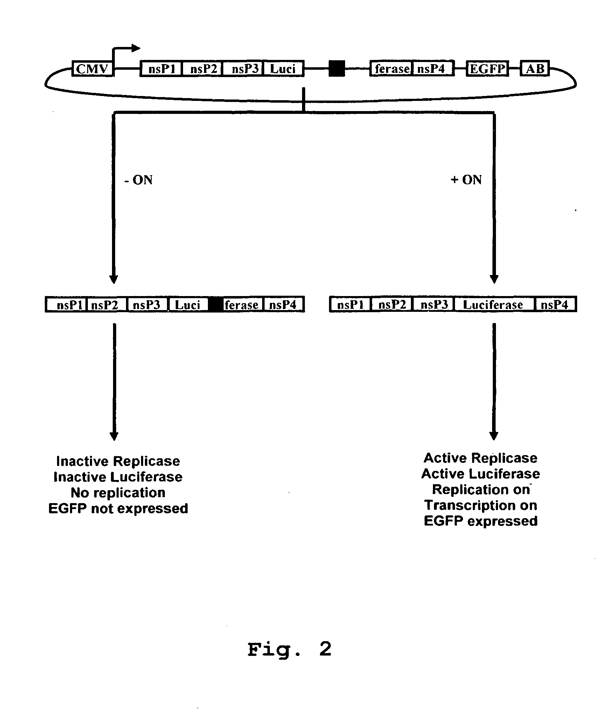

Fig. 2 exemplifies the splice-switch of a layered vector, here pCMV-SFV1-Luc6+7. The circular drawing is a schematic representation of the layered vector. Boxes represent "exons" and heavy lines represent introns. The box with an arrow represents the promoter (here in this example human cytomegalovirus immediately early promoter), under which is expressed the RNA corresponding to the genome of the replicon vector and used for translation of the following proteins: nsP1, nsP2, nsP3, Luciferase and nsP4. The nsP1, nsP2, nsP3 and nsP4 are from viral origin and represent the replicase of SFV. The EGFP is expressed in a co-replication manner and is used here as a marker of successful replication and transcription. The region corresponding to the luciferase gene contains the intron responsible for the aberrant splicing. The luciferase activity will be used as a quantitative marker of correct splicing of pre-mRNA and its subsequent replication. The black boxes represent the portion of intron that remains in the aberrantly spliced mRNA molecule. Once present in a mature mRNA it is responsible for the inactivation of replication and the absence of green fluorescence of the cells. In the absence of any splice rescuing oligonucleotide (-ON), the aberrant splicing is not reversed and a portion of the intronic sequence is included to the luciferase mRNA and causes a frame shift that renders inactive any luciferase activity. In the case of the presence of any splice rescuing oligonucleotide (+ON), the luciferase is active, replication is possible and EGFP is expressed.

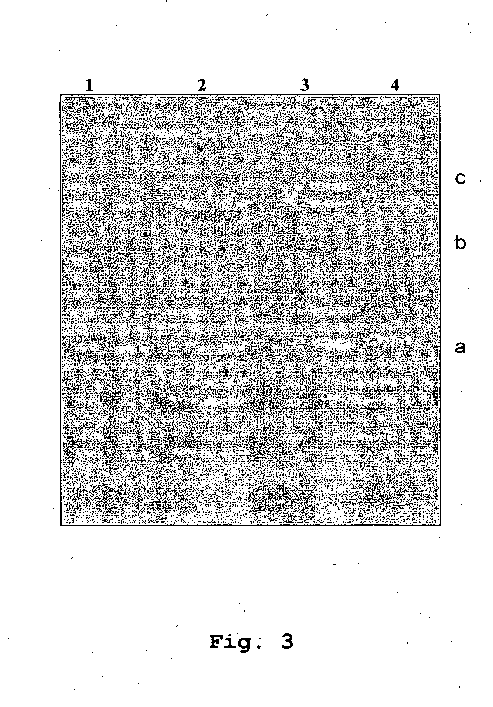

Fig. 3 shows the reversal of aberrant splicing of the artificial intron, containing two mutations, inside the pCMV-SFV1-Luc6+7 construct when transfected to HeLa cells. Lane 1 represents the intron's aberrant splicing in the absence of splice-switch oligonucleotide. Lanes 2, 3 and 4 represent the splicing when the splice switch oligonucleotide 654 (SS654), the splice switch oligonucleotide 705 (SS705) or a cocktail of both (SS654 and SS705) are respectively present in the cells.

The bands were obtained from RT-PCR with primers designed to amplify specifically the intron. Band (a) represents the correctly spliced intron. Bands (b) and (c) represent the aberrantly spliced mRNAs and are only present in lanes 1, 2 and 3. The intensity of bands (a) increase from lane 1 to lane 4 showing that when the cocktail of two oligonucleotides (SS654 and SS705) is provided, then the correct splicing of the artificial intron containing two mutations is completely rescued.

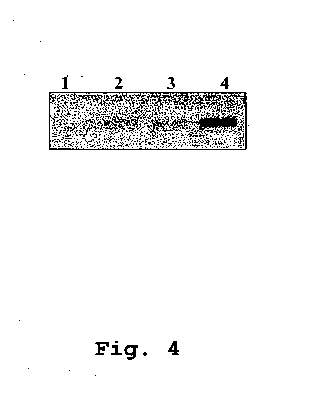

Fig. 4 shows the reversal of aberrant splicing of the artificial intron, containing two mutations, inside the AdenoLuc6+7 construct when transfected to HeLa cells.

Lane 1 represents the intron's aberrant splicing in the absence of splice-switch oligonucleotide. Lanes 2, 3 and 4 represent the splicing when oligonucleotide SS654, SS705 or a cocktail of both are respectively present in the cells.

The bands were obtained from RT-PCR with primers designed to amplify specifically the intron and represent the correctly spliced intron. The intensity of the bands increase from lane 1 to lane 4 showing the efficiency of the splice-switch oligonucleotides rescuing the correct splicing. When the cocktail of two oligonucleotides (SS654 and SS705) is provided (lane 4) then the splicing of the artificial intron containing two mutations is more efficient in this case than either one oligonucleotide or the other alone.

### BEST EMBODIMENTS FOR CARRYING OUT THE INVENTION

### Example 1

Demonstration of the inhibitory effect caused by one splice-defective intron with one mutation responsible for an aberrant splicing of the corresponding pre-mRNA molecule in HeLa cells on the infectivity of the layered alphavirus replicon vector.

The vector used for this example is based on the layered SFV replicon vector containing an insertion of firefly luciferase gene between regions, encoding replicase proteins nsP3 and nsP4 designated as pCMV-SFVl-Luc-EGFP (SEQ.ID.NO 1). Intron which is the wild type (wt) second intron of human beta-globin gene (SEQ.ID.NO 2), the same intron with T to G substitution at position 705 (SEQ.ID.NO 3) or with C to T substitution at position 654 (SEQ.ID.NO 4) or the first intron from human beta-globin gene with G to A substitution at position 110 (SEQ.ID.NO 5) is inserted inside of the luciferase gene resulting vectors pCMV-SFV1-Lucwt (SEQ.ID.NO 6), pCMV-SFV1-Luc705 (SEQ.ID.NO 7), pCMV-SFV1-Luc654 (SEQ.ID.NO 8) or pCMV-SFV1-Luc110 (SEQ.ID.NO 9), respectively. Thus, when correctly spliced, the rescued genome is capable of replication and serves as template for luciferase expression. In contrast, when aberrantly spliced, the reading frame of the replicase is interrupted and the resulting RNA can not replicate (does not produce polymerase) nor does it serve as a template for luciferase expression (due to the interrupted reading frame) and does not activate expression of inserted EGFP marker (Figure 2). It is therefore possible to measure in a quantitative way the luciferase activity of the HeLa cells transfected with the SFV constructs; and indirectly the efficiency of the defective intron to cause aberrant splicing.

For each construct total amount of 2µg of layered vector DNA was used for transfection of 500 000 HeLa cells using Lipofectamine 2000 (Invitrogen) according to manufacturer's protocol. Luciferase activity was measured 24 h post-transfection. Results shown in Table 1 are normalized to the amount of luciferase, expressed by control vector, which has been taken as 100%

**Table 1.**

| | Percentage of Relative Luciferase Activity (RLA) |
|---|---|
| pCMV-SFV1-Lucwt | 100 |
| pCMV-SFV1-Luc705 | 70 |
| pCMV-SFV1-Luc654 | 57 |
| pCMV-SFV1-Luc110 | <100 |

Analysis of these data shows that a wt intron can be inserted to the sequence of a layered vector and this last one remains active indicating correct and efficient removal of wt intron from transcripts, synthesized in cell nucleus. Furthermore, the insertion of a defective intron 705, intron 654 or intron 110 instead of wt intron is effective in reducing the luciferase activity produced by the vector and, accordingly, the efficiency of its rescue from the plasmid vector: the decrease in RLA observed between wt-control and constructs with mutant introns can only result from aberrant splicing of the pre-mRNA molecule issued from the vectors with the mutant intron. This aberrant splicing fails to remove intron completely and, as consequence, results in lethal mutation of the RNA genome of alphavirus vector.

Conclusion. Insertion of a single aberrantly spliced intron into SFV layered replicon vector substantially reduces its infectivity. Aberrantly spliced introns from different origin and with completely different sequences produced the same effect. Introns with the same basic sequence but containing different point mutations caused similar effects. Accordingly, this data supports our claim that any naturally occurring aberrantly spliced intron can be used in this invention.

### Example 2

Demonstration of the inhibitory effect caused by single splice-defective intron of artificial origin responsible for an aberrant splicing of corresponding pre-mRNA molecule in HeLa cells on the infectivity of the layered alphavirus replicon vector.

The previous example covers the use of all naturally occurring aberrantly spliced introns used in order to suppress the efficiency of rescue of alphavirus replicon form layered vectors. To demonstrate the feasibility of the method for aberrantly spliced introns of artificial origin (any intron, artificially modified at any way or designed *in silico*) an artificial intron containing combination of two mutations, responsible, respectively, for human beta-thalassaemia in its Mediterranean version (mutation T to G at position 705 of the intron) and its Chinese version (mutation C to T at position 654 of the intron) was designed and constructed. Thus, this intron presents mutations that do not co-exist in nature and will be referred as intron 6+7. Full sequence of the intron 6+7 disclosed as SEQ.ID.NO 10.

The artificial intron was inserted in the SFV layered replicon vector at the same way as 654 or 705 introns; the vector was designated as pCMV-SFV1-Luc6+7, its sequence is disclosed as SEQ.ID.NO 11. The vector pCMV-SFV1-Luc6+7 was assayed by the method described in example 1. The results of this analysis are presented in Table 2.

**Table 2.**

| | Percentage of Relative Luciferase Activity (RLA) |
|---|---|
| pCMV-SFV1-Lucwt | 100 |
| pCMV-SFV1-Luc6+7 | 32 |

Analysis of these data shows that the insertion of a defective artificial intron 6+7 effectively reduce the luciferase activity (and, correspondingly, the efficiency of the replicon vector genome rescue) of the sample. It also revealed that the inhibition was much stronger than observed for naturally occurring aberrantly spliced intron presenting a single mutation 705 or 654 (table 1).

Conclusion. Artificially designed introns can be used in this invention. As revealed by this example, such introns may have stronger effects on the blocking of the rescue of the genome of the replicon vector than the naturally occurring aberrantly spliced introns. It also shows that artificial introns can be constructed by combining naturally occurring mutations into a single intron; the numbers of these mutations are not restricted to two. It is also envisioned that any artificial intron, either derived from the natural intron by introduction of one or several mutations or designed in any other way (up to completely artificial, e.g. *in silico* designed sequence) can be used in this invention.

### Example 3

Demonstration of the inhibitory effect caused by two defective introns responsible for aberrant splicing on the rescue of layered replicon vector genome in HeLa cells.

Examples 1 and 2 revealed the usability of an aberrantly spliced intron for the suppression of the rescue of replicon RNA genome from the corresponding alphavirus layered vector. It also demonstrated that the magnitude of suppression is not very high in case of using natural introns and even in case of artificially designed intron. Most probably it reflects the fact that few or even a single correctly spliced transcript (most, if not all, aberrantly spliced introns have also some background level of correct splicing as well; in other words, splicing defect is generally not an absolute one) can initiate the replication of alphavirus vector and the effects downstream of this event are not affected by the aberrantly spliced intron any longer. Accordingly, the efficiency of suppression of the viral (or replicon vector) genome rescue should be increased. One approach to achieve this goal is disclosed in example 2 - it is the use of a more efficient (less frequently correctly spliced) intron which can also be an intron of artificial design. An alternative would be the use of more than one aberrantly spliced intron in the viral part of the layered vector.

The vector used for this example is based on the layered SFV replicon vector pCMV-SFV1-Luc-EGFP. Two sites were used for intron insertion - the region corresponding to inserted luciferase gene and the region, corresponding to the region encoding nsP4 replicase protein. To prove the efficiency of this approach four constructs were designed and constructed. Control vector pCMV-SFV1-Lucwt/wt (SEQ.ID.NO 12) contained wt introns (the second intron for human beta-globin gene) in both positions. The second construct, pCMV-SFV1-Luc705/705 (SEQ.ID.NO 13) contained two identical aberrantly spliced introns (the second intron from human beta globin with T to G substitution at position 705). The third vector pCMV-SFV1-Luc705/654 (SEQ.ID.NO 14) contained an intron with T to G substitution at position 705 inside the region corresponding to the luciferase gene and intron with C to T substitution at position 654 in the region corresponding to nsP4 (thus, the construct contained two different naturally occurring aberrantly spliced introns). Finally, the fourth vector pCMV-SFV1-Luc6+7/6+7 (SEQ.ID.NO 15) contained two identical aberrantly spliced artificial introns (the second intron from human beta globin with point mutations in positions 654 and 705, referred as intron 6+7). The constructs were assayed as described in Example 1; the results of the analysis are shown in Table 3.

**Table 3**

| | Percentage of Relative Luciferase Activity (RLA) |
|---|---|
| pCMV-SFV1-Lucwt/wt | 100 |
| pCMV-SFV1-Luc705/705 | 5 |
| pCMV-SFV1-Luc705/654 | 5 |
| pCMV-SFV1-Luc6+7/6+7 | 2 |

Analysis of these data shows that two wt introns can be inserted to the sequence of a layered vector and this last one remains fully active indicating correct and efficient removal of both wt introns from transcripts, synthesized in cell nucleus. Furthermore, the insertion of two defective introns is effective in reducing the luciferase activity of the sample (and, correspondingly, the rescue of the infectious replicon genomes). Importantly, the presence of two defective introns results in a much stronger effect than the presence of single aberrantly spliced intron and this effect is even stronger when each intron contains more than one mutation.

Conclusions. Data presented in this example indicates that more than one intron can be introduced into an alphavirus layered vector. Ultimately it indicates that any number of introns (limited only by the size of the construct and practical considerations) can be introduced into any viral vector. In case of the use of aberrantly spliced introns, cumulative effect on the rescue and replication of the replicon vector was observed. These aberrantly spliced introns may be identical to each other or may be different; they may be both of natural and/or artificial origin. By increasing the number of inserted introns it is possible to achieve complete blockage of the rescue of infectious replicon RNA or complete block of gene expression (and, consequently, replication) of DNA genomic virus.

### Example 4

Reversal of aberrant splicing and activation of rescue of an alphavirus replicon vector by antisense oligonucleotides targeting pre-mRNA molecules of the vector.

Technology exemplified in Examples 1-3 has little or no practical value unless there is a method to reverse the effect caused by the insertion of aberrantly spliced intron or introns. The greatest value of the technology would be achieved if this reversion might be achieved in specific cells. The method to reverse the suppression of replicon vector rescue is presented in this example. This method includes the application of specific composition consisting of antisense oligonucleotides, Splice Switch oligonucleotide 705 (SS705, SEQ.ID.NO 16) and Splice Switch oligonucleotide 654 (SS654, SEQ.ID.NO 17) to the cell in order to restore correct splicing.

All the constructs designed with one or more defective introns and detailed in Examples 1 to 3 produced pre-mRNA molecules that led to aberrant splicing events and ultimately to a drastic decrease in RLA in the samples. All these constructs were assayed in order to reveal if the presence of the specific compositions of splice-switch oligonucleotide (or oligonucleotides) does rescue the infectivity of the corresponding layered replicon vector. To assay this, the HeLa cells were transfected (control cells were mock-transfected) with composition consisting of antisense oligonucleotide or oligonucleotides at final concentration 100 nM and RNAiMAX (Invitrogen) transfection reagent or other transfection reagent(s). 24 h later cells were transfected with layered vectors and analyzed as described in Example 1. Closely similar results were obtained for all vectors; therefore only those for the vector pCMV-SFV1-Luc6+7/6+7 (vector containing two artificial introns) are shown in Table 4.

**Table 4**

| Vector | Transfection with oligonucleotide | Percentage of Relative Luciferase Activity (RLA)* |
|---|---|---|
| pCMV-SFV1-Luc6+7/6+7 | Mock | 2 |
| pCMV-SFV1-Luc6+7/6+7 | SS705 | 8 |
| pCMV-SFV1-Luc6+7/6+7 | SS654 | 11 |
| pCMV-SFV1-Luc6+7/6+7 | SS654 +SS705 | 40 |

| | | |
|---|---|---|
| * normalized to the vector with two wild type introns It was demonstrated by using reverse-transcription PCR technology that the increase of the luciferase activity and thus the efficiency of rescue of infectious (replicating) genomes was achieved by correction of the aberrant splicing (Figure 3) and does not represent any accidental effect caused by the presence of the used composition. It was further demonstrated that the increase in concentration of the antisense oligonucleotides, optimization of their relation and the improvement of the delivery methods allowed to achieve up to 100% recovery of the infectivity of the layered replicon vector (thus, completely eliminating the effect caused by the insertion of aberrantly spliced introns). | | |

Conclusion. Delivery of the composition consisting of splice-switch oligonucleotides to the cells efficiently rescues the infectivity of layered alphavirus replicon vector. The rescue is achieve by blocking the aberrant splicing and thus forcing splicing back to normal pathway (Figure 3); thus the rescue does not represent unspecific effect caused by component or components of composition such as transfection reagent and oligonucleotide(s) or by transfection procedure. The efficiency of the rescue depends on the type of antisense oligonuclotide; it also depends on the amount of oligonucleotide in the composition and the method of its delivery. By selecting optimal conditions, full reversion of the lethal phenotype caused by the insertion of aberrantly spliced introns can be achieved. Some antisense oligonucleotides were observed to be more efficient in the rescue of the infectivity of the construct than others (SS654 was always more efficient that SS705), an effect which can be explained by the "enhancement of splicing" phenomenon. To revert the phenotype of the vector containing introns with more than one point mutation (or combination of different introns) a composition containing a cocktail of antisense oligonucleotides was required. It is envisioned that the composition of the cocktail may include not only splice-switch oligonucleotides but also oligonucleotides with the ability to cause enhancement of correct splicing.

### Example 5

Demonstration of the inhibitory effect caused by two defective introns responsible for aberrant splicing on the rescue of layered genomic alphavirus vector genome in HeLa cells and reversion of this effect by using composition containing antisense splice-switch oligonucleotides. Examples 1-4 revealed the usability of an aberrantly spliced intron for suppression of the rescue of replicon RNA genome from the layered vector and the ability of composition containing antisense splice-switch oligonucleotides to reverse this effect. However, since the replicon vectors have limited use in anti-cancer therapy (as they are restricted to a single cell and do not spread from infected cell to neighbouring cells) it should be demonstrated that the same effects can be reproduced in the case of layered genomic vectors of alphaviruses. In this case the efficiency of suppression of viral rescue is even more crucial, since even a single event of production of correctly spliced RNA would result in the replication of the virus in transfected cells and its spread in cell culture (or in case of in vivo application in tissue and organism). Therefore the constructs with more than one aberrantly spliced intron in the viral part of a layered vector were constructed on the bases of layered SFV genomic vector pCMV-SFV4-Luc (SEQ.ID.NO 18) containing the insertion of firefly luciferase gene between regions encoding replicase proteins nsP3 and nsP4. The same vector also contained a native wild type intron from rabbit beta-globin gene inserted into the region corresponding to the structural proteins of the virus (it was made in order to stabilize the plasmid and increase infectivity; WO2009/033490, "A method for creating a viral genomic library, a viral genomic library and a kit for creating the same", Rausalu et al.). Two additional sites were used for the insertion of the intron - the region corresponding to inserted luciferase gene and the region corresponding to the region encoding for nsP4 replicase protein. To prove the efficiency of this approach, four constructs were designed and constructed. The control vector, referred as pCMV-SFV4-Lucwt/wt (SEQ.ID.NO 19), contained wt introns (the second intron for human beta-globin gene) in both positions. The second pCMV-SFV4-Luc705/705 (SEQ.ID.NO 20) construct contained two identical aberrantly spliced introns (the second intron from human beta globin gene with T to G substitution at position 705). The third vector pCMV-SFV4-Luc705/654 (SEQ.ID.NO 21) contained an intron with the T to G substitution at position 705 inside the region corresponding to the luciferase gene and an intron with C to T substitution at position 654 in the region, corresponding to nsP4 (thus, the construct contained two different naturally occurring aberrantly spliced introns). Finally, the fourth vector pCMV-SFV4-Luc6+7/6+7 (SEQ.ID.NO 22) contained two identical aberrantly spliced artificial introns 6+7 (the second intron from human beta globin gene with point mutation in position 654 and 705). The constructs were:
1. transfected to BHK21 cells by electroporation and assayed for infectivity by the use of infectious centre assay.
2. transfected to HeLa cells by electroporation and assayed for infectivity by the use of infectious centre assay.
3. transfected to HeLa cells by use of various means of transfection (including but not limited to electroporation and lipofection) and assayed for infectivity by measuring the luciferase activity.

The results are presented in Table 5; all values are normalized to the values produced by the vector with wt introns (pCMV-SFV4-Lucwt/wt) taken as 100%

**Table 5**

| | Percentage of plaques on BHK 21 cells. | Percentage of plaques on HeLa cells. | Percentage of Relative Luciferase Activity (RLA) in HeLa cells. |
|---|---|---|---|
| pCMV-SFV4-Lucwt/wt | 100 | 100 | 100 |
| pCMV-SFV4-Luc705/705 | 10 | Below detection limit | 5 |
| pCMV-SFV4-Luc706/654 | 11 | Below detection limit | 12 |
| pCMV-SFV4-Luc6+7/6+7 | 2 | Below detection limit | 1 |

Analysis of this data shows that two additional wt introns can be inserted to the sequence of a layered genomic vector pCMV-SFV4-Luc and this last one remains fully active indicating correct and efficient removal of both wt introns from transcripts, synthesized in the cell nucleus. Furthermore, the insertion of two defective introns is effective in reducing the luciferase activity of the sample and the rescue of the infectious virions. Importantly, the presence of two defective introns results in a much stronger (cumulative) effect and this effect is even stronger when each intron contains more than one mutation.

As with layered replicon vectors, the technology exemplified above has no practical value unless the effect caused by the insertion of aberrantly spliced intron or introns can be reversed. Therefore the method based on the application of specific composition containing antisense splice-switch oligonucleotides to the cell in order to restore correct splicing was tested also on the layered genomic vectors, oligonucleotides SS705 and SS654 (sequences disclosed as SEQ.ID.NO 16 and SEQ.ID.NO 17 respectively) were used as part of the composition in this assay. To assay this, the HeLa cells were transfected (control cells were mock-transfected) with the composition containing antisense oligonucleotide or with the combination of antisense oligonucleotides at the final concentration of 100 nM and transfection reagent RNAiMAX (Invitrogen) or other transfection reagent(s). 24 h later the cells were transfected with layered genomic vectors and analyzed 24 h later for luciferase activity (which, as shown above, is in good correlation with the release of infectious virus progeny). Results of this experiment are presented in Table 6.

**Table 6**

| | Percentage of Relative Luciferase Activity (RLA) in cells in the absence of the composition | Percentage of Relative Luciferase Activity (RLA) in cells, transfected with the composition of appropriate oligonucleotide or oligonucleotides (in parenthesis). | Efficiency of rescue by the composition containing antisense splice-switch oligonucleotide or oligonucleotides |
|---|---|---|---|
| pCMV-SFV4-Lucwt/wt | 100 | 100 (none) | Not applicable |
| pCMV-SFV4-Luc705/705 | 5 | 90 (SS705) | Ca 20 fold |
| pCMV-SFV4-Luc705/654 | 12 | 100 (SS705+SS654) | Ca 10 fold |
| pCMV-SFV4-Luc6+7/6+7 | <1 | 100 (SS705+SS654) | >100 fold |

Thus, similarly to the system based on the use of layered replicon vectors, the infectivity of layered genomic vectors of SFV was efficiently rescued by composition containing the splice-switch oligonucleotide(s) and transfection reagent. The same trends as for the replicon vectors were observed (see Example 4, Table 4). The infectivity of the constructs was fully restored by using a composition containing a cocktail of splice-switch oligonucleotides with proper concentration and sequences. This supports the conclusion that even the most extensive defects, caused by the insertion of aberrantly spliced introns (even if inserted in bigger numbers and/or in forms, more efficiently suppressing the correct splicing) could be reverted by the presence of properly formulated composition containing cocktail of splice-switch oligonucleotides and transfection reagent or system.

It should be emphasized that in the case of layered genomic vectors, aberrantly spliced introns should be inserted into areas corresponding to the region encoding viral replicase. Insertion of aberrantly spliced introns into areas, corresponding to the region encoding viral structural proteins would not block the expression of replicase proteins and thus will allow such genomes to replicate. In such genomes the reading frame of the structural proteins will be interrupted by the remains of the incorrectly spliced introns making it impossible to express structural proteins and form new virions, however, due to the high mutation rate of viruses with RNA genomes these defects can (at least in theory) be reverted during replication resulting in infectious viral progeny.

Conclusions. These data indicate that the described approaches and technologies are equally suitable for layered replicon vectors as well as for layered genomic vectors of positive strand RNA viruses.

### Example 6

Construction of vectors based on DNA viruses containing defective intron(s) responsible for the aberrant splicing of pre-mRNAs expressed by these vectors and demonstration of the rescue of this defect by composition of antisense splice-switch oligonucleotides.

The vector used for this example is based on an Adenovirus which has double stranded linear DNA genome. Adenovirus is also envisioned as one of the most promising anti-cancer vectors among all viruses with DNA genomes. Ability to regulate gene expression in adenovirus vector is directly linked with ability to regulate virus replication (if one can regulate gene expression one can also regulate replication). Adeasy system (Stratagene) was used to construct the adenovirus vectors, where native E1A/E1B region of the virus was substituted with luciferase gene placed under the control of cytomegalovirus immediately early promoter. The vector was designed so that it expresses a firefly luciferase when corresponding pre-mRNA is correctly spliced. It is therefore possible to measure in a quantitative way the luciferase activity of the HeLa cells transfected with the adenovirus construct. The second intron from human beta-globin gene, inserted into the vector designated as Adeno-Lucwt (SEQ.ID.NO 23) contains no mutation and will be referred to as wt; the intron inserted into the vector designated as Adeno-Luc705 (SEQ.ID.NO 24) represents the second intron from human beta-globin gene containing the substitution T to G at position 705 as it is found in case of human beta-thalassaemia in its Mediterranean version; the intron inserted into the vector designated as Adeno-Luc6+7 (SEQ.ID.NO 25) is an artificial intron 6+7, which originates from the second intron of human beta-globin gene where two point mutations, T to G in position 705 and C to T in position 654 have been introduced. It is assumed that any combination of these and/or other introns could be introduced to the genome of any adenovirus or adenovirus-based vector; the provided constructs represent sufficient example to illustrate the application of this invention in case of adenovirus. Since the luciferase gene with the intron is inserted in the region which in the native adenovirus contains the expression unit for E1A/E1B proteins, the effect of the defective intron on the luciferase marker expression will reflect the effect of the insertion of a similar intron on the expression of the viral trans-activator (E1A) and the anti-apoptotic (E1B) proteins.

Experiments were performed as followed: mock-transfected HeLa cells were used as controls and HeLa cells transfected with composition containing the appropriate oligonucleotide or oligonucleotides were used as experimental cells. 24 hours post transfection both experimental and control cells were transfected with adenoviruses and the luciferase activities were determined in 24 hours post transfection. Results of this experiment are shown in Table 7 (activities are normalized to the control vector which contained wt intron; luciferase activity produced by this vector was taken as 100%)

**Table 7**

| | Percentage of Relative Luciferase Activity (RLA) in the absence of the composition containing splice-switch oligonucleotide | Percentage of Relative Luciferase Activity (RLA) in the presence of the composition containing corresponding splice-switch oligonucleotide or oligonucleotides (identity shown in parenthesis) |
|---|---|---|
| Adeno-Lucwt | 100 | 100 |
| Adeno-Luc705 | 8 | 56 (SS705) |
| Adeno-Luc6+7 | 3 | 61 (SS705+SS654) |

This data shows that introns can be inserted to the sequence of a DNA virus or viral vector based on such virus. Furthermore, the insertion of a defective intron 705 is highly effective in reducing the luciferase activity of the sample (12.5 fold reduction). These results are coherent with the data obtained from the previous examples. The insertion of a defective intron 6+7 is even more effective in reducing the luciferase activity of the sample (over 30 fold). Also the relative effect of insertion of aberrantly spliced introns into adenovirus genomes is more prominent than the effects caused by the same introns in layered alphavirus vectors (compare present data with those from Table 1 and 2). This can be explained by the fact that in the case of layered alphavirus vectors, only the rescue of self-replicating RNAs (replicons or genomes) is inhibited whereas the whole gene expression and consequently the whole replication cycle will be inhibited in the case of adenoviruses and other viruses with DNA genomes. Furthermore, all results obtained with the adenovirus system are highly coherent with those obtained with the layered vectors of alphaviruses indicating that all main conclusions, made on the bases of Examples 1-5 are also correct for adenoviruses and other DNA genomic viruses replicating in nucleus of infected cells. The ability of the composition of splice-switch oligonucleotides to activate luciferase expression of Adeno-Luc-705 (7-fold activation) and Adeno-Luc-6+7 (over 20-fold activation) demonstrate the reversion of the introduced defect. It was confirmed by reverse-transcription PCR analysis that this reversion occurs due to the splice-switch activity of the oligonucleotides used in the composition (Figure 4) and does not represent any unspecific side effect of an oligonucleotide, transfection reagent or both.

Conclusions. These data allow concluding that the suppression of gene expression and replication of the viruses with DNA genomes replicating in the nucleus of infected cells is at least as efficient as it is in case of layered vectors of positive strand RNA viruses (and likely even more). This artificially caused defect can be reverted by providing the composition containing splice-switch antisense oligonucleotide(s). Therefore, it can be concluded that the developed technology can be efficiently used for all viruses belonging to these groups as well as for vectors based on these viruses.

## Claims

1. A composition for use in elimination of the viability of a eukaryotic cell by simultaneous or consecutive introduction of its components (a) and (b) into a eukaryotic cell, said composition comprising (a) a cytolytic virus or a vector originating from a cytolytic virus with one or more eukaryotic intron(s) introduced, which intron(s) comprise(s) one or more mutations interfering with correct removal of said intron(s) by naturally occurring splicing processes, and wherein the insertion of said intron(s) interrupts the expression of a viral component with a vital function and results in a lethal phenotype of said virus or vector, and (b) oligonucleotides, modified oligonucleotides or oligonucleotide analogues specifically able to restore the correct splicing of the introduced eukaryotic intron(s).

2. The composition of Claim 1, wherein the virus is a DNA genomic virus.

3. The composition of Claim 1, wherein the vector is a layered vector containing complementary DNA of an RNA-virus or a construct originating from an RNA virus.

4. The composition of Claim 1, wherein the virus is an alphavirus or a vector based on an alphavirus.

5. The composition of Claim 4, wherein the virus is Semliki Forest virus.

6. The composition of Claim 1, wherein the virus is an adenovirus or a vector based on an adenovirus.

7. The composition of Claim 1, wherein the intron has a naturally occurring nucleotide sequence.

8. The composition of Claim 1, wherein the nucleotide sequence of the intron is modified.

9. The composition of Claim 1, wherein the nucleotide sequence of the intron is artificially generated.

10. The composition of Claim 1, wherein the introduced intron is the second intron of human beta-globin gene with T to G substitution at position 705 or with C to T substitution at position 654.

11. The composition of Claim 1, wherein the introduced intron is the second intron of human beta-globin gene with T to G substitution at position 705 and with C to T substitution at position 654.

12. The composition of Claim 1, wherein the eukaryotic cell is a neoplastic, tumour or cancer cell.

13. The composition of Claim 1 for use in the treatment or prevention of neoplasms.

14. The composition of Claim 1, or the composition of Claim 1 for the use as recited in Claim 13, for use in human medicine.

15. The composition of Claim 1, or the composition of Claim 1 for the use as recited in Claim 13, for use in veterinary medicine.

## Patentansprüche

1. Zusammensetzung für die Eliminierung der Lebensfähigkeit einer eukaryotischen Zelle durch eine gleichzeitige oder konsekutive Einführung ihrer Komponenten (a) und (b) in die eukaryotische Zelle, die genannte Zusammensetzung enthält (a) einen zytolytischen Virus oder einen Vektor, der von einem zytolytischen Virus stammt dem ein oder mehrere eukaryotische Intron(s) eigeführt wurden, wessen Intron(s) eine oder mehrere Mutationen beinhalten, die bei der korrekten Entfernen des/der genannten Intron(s) durch natürlich auftretende Splicing-Prozesse eingreifen, und **dadurch gekennzeichnet, dass** die Einführung des/der genannten Intron(s) die Expression einer viralen Komponente mit einer vitalen Funktion unterbricht und zu einem letalen Phänotyp des genannten Virus oder Vektors führt, und (b) Oligonukleotide, modifizierte Oligonukleotide oder Oligonukleotidanaloga, die besonders befähigt sind, das korrekte Splicing des/der eingeführten eukaryotischen Introns wiederherzustellen.

2. Zusammensetzung nach Anspruch 1, **dadurch gekennzeichnet, dass** der Virus ein genomischer DNA Virus ist.

3. Zusammensetzung nach Anspruch 1, **dadurch gekennzeichnet, dass** der Vektor ein geschichteter Vektor ist der die komplementäre DNA eines RNA-Virus oder eine, von einem RNA-Virus stammendes Konstrukt enthält.

4. Zusammensetzung nach Anspruch 1, **dadurch gekennzeichnet, dass** der Virus ein Alphavirus oder ein auf einen Alphavirus basierender Vektor ist.

5. Zusammensetzung nach Anspruch 4, **dadurch gekennzeichnet, dass** der Virus ein Semliki-Forest-Virus ist.

6. Zusammensetzung nach Anspruch 1, **dadurch gekennzeichnet, dass** der Virus ein Adenovirus oder ein auf einem Adenovirus basierender Vektor ist.

7. Zusammensetzung nach Anspruch 1, **dadurch gekennzeichnet, dass** das Intron eine natürlich auftretende Nukleotidsequenz hat.

8. Zusammensetzung nach Anspruch 1, **dadurch gekennzeichnet, dass** die Nukleotidsequenz des Introns modifiziert ist.

9. Zusammensetzung nach Anspruch 1, **dadurch gekennzeichnet, dass** die NukleotidSequenz des Introns künstlich generiert ist.

10. Zusammensetzung nach Anspruch 1, **dadurch gekennzeichnet, dass** das eingeführte Intron das zweite Intron des menschlichen β-Globin-Gens ist mit T zu G Substitution an Position 705 oder mit C zu T Substitution an Position 654.

11. Zusammensetzung nach Anspruch 1, **dadurch gekennzeichnet, dass** das eingeführte Intron das zweite Intron des menschlichen β-Globin-Gens ist mit T zu G Substitution an Position 705 oder mit C zu T Substitution an Position 654.

12. Zusammensetzung nach Anspruch 1, **dadurch gekennzeichnet, dass** die eukaryotische Zelle eine neoplastische, Tumor- oder Krebszelle ist.

13. Zusammensetzung nach Anspruch 1 zur Verwendung in der Behandlung oder Vorbeugung von Blastomen.

14. Zusammensetzung nach Anspruch 1, oder Zusammensetzung nach Anspruch 1 für die Verwendung wie in Anspruch 13 erwähnt, zur Verwendung in der Humanmedizin.

15. Zusammensetzung nach Anspruch 1, oder die Zusammensetzung nach Anspruch 1 für die Verwendung wie in Anspruch 13 erwähnt, zur Verwendung in der Veterinärmedizin.

## Revendications

1. Composition pour éliminer la viabilité d'une cellule eucaryote par l'introduction simultanée ou consécutive de ses composants (a) et (b) dans une cellule eucaryote, ladite composition comprenant (a) un virus ou vecteur cytolytique provenant d'un virus cytolytique avec un ou plusieurs intron(s) eucaryote introduits, dont l'intron(s) comporte(nt) une ou plusieurs mutations interférant avec l'élimination correcte dudit intron(s) par des processus d'épissage d'origine naturelle, et dans lequell' insertion dudit intron(s) interrompt l'expression d'un composant viral avec une fonction vitaleet conduit à un phénotype létal dudit virus ou vecteur, et (b) des oligonucléotides, des oligonucléotides modifiés ou des analogues des oligonucléotides spécifiquement capables àre staurer l'épissage correct de l'intron(s) eucaryote introduit(s).

2. Composition selon la revendication 1, dans laquelle le virus est un virus d'ADN génomique.

3. Composition selon la revendication 1, dans laquelle le vecteur est un vecteur en couches contenant d'un ADN complémentaire de virus ARN ou une construction provenant d'un virus ARN.

4. Composition selon la revendication 1, dans laquelle le virus est un alphavirus ou un vecteur basé à un alphavirus.

5. Composition selon la revendication 4, dans laquelle le virus est le virus de Semliki Forest.

6. Composition selon la revendication 1, dans laquelle le virus est un adénovirus ou un vecteur basé sur un adénovirus.

7. Composition selon la revendication 1, dans laquelle l'intron a une séquence de nucléotides d'origine naturelle.

8. Composition selon la revendication 1, dans laquelle la séquence de nucléotides de l'intron est modifié.

9. Composition selon la revendication 1, dans laquelle la séquence de nucléotides de l'intron est générée de manière artificielle.

10. Composition selon la revendication 1, dans laquelle l'intron introduit est le second intron du gène de la bêta-globine humaine avec la substitution de T à G à la position 705 ou avec la substitution de C à T à la position 654.

11. Composition selon la revendication 1, dans laquelle l'intron introduit est le second intron du gène de la bêta-globine humaine avec la substitution de T à G à la position 705 et avec la substitution de C à T à la position 654.

12. Composition selon la revendication 1, dans laquelle la cellule eucaryote est une cellule néoplasique, une tumeur ou un cancer.

13. Composition selon la revendication 1 destinée à l'utilisation pour le traitement ou la prévention des néoplasmes.

14. Composition selon la revendication 1, ou la composition selon la revendication 1 pour l'utilisation selon la revendication 13, destinée pour l'utilisation en médecine humaine.

15. Composition selon la revendication 1, ou la composition selon la revendication 1 pour l'utilisation selon la revendication 13, destinée pour l'utilisation en médecine vétérinaire.
